# EUROPEAN PATENT SPECIFICATION

(11) **EP 2 488 516 B1**
(45) Date of publication and mention of the grant of the patent: **01.04.2015**
(21) Application number: 10809247.9
(22) Date of filing: 13.10.2010
(51) Int. Cl.: C07D 411/04

(54) **PROCESS FOR THE PREPARATION OF LAMIVUDINE AND NOVEL SALTS IN THE MANUFACTURE THEREOF**
VERFAHREN ZUR HERSTELLUNG VON LAMIVUDIN UND NEUE SALZE AUS SEINER HERSTELLUNG
PROCÉDÉ POUR LA PRÉPARATION DE LAMIVUDINE ET NOUVEAUX SELS DANS SA FABRICATION

(30) Priority: 14.10.2009 IN CH24922009
(43) Date of publication of application: 22.08.2012
(73) Proprietor: MYLAN LABORATORIES LIMITED, 500033 Andhra Pradesh (IN)
(72) Inventor: VELLANKI, Sivaram, Prasad, Hyderabad 500 055 (IN); SAHU, Arabinda, Hyderabad 500 055 (IN); BALUSU, Rajababu, Hyderabad 500 055 (IN); PHADURI, Naveen, Kumar, Hyderabad 500 055 (IN); DEEVI, Venkata, Srimannarayana, Hyderabad 500 055 (IN); KILARU, Ravindrababu, Hyderabad 500 055 (IN); ANNADASU, Ankama, Nayudu, Hyderabad 500 055 (IN); DATTA, Debashish, Hyderabad 500 055 (IN)
(74) Representative: Gillard, Richard Edward
(86) International application number: PCT/IN2010/000679
(87) International publication number: WO 2011/045815

(56) References cited:
- WO-A1-2007/119248
- WO-A1-2009/069013
- WO-A2-2008/114279
- WO-A2-2009/037538
- HARRIS R K ET AL: "Polymorphism in a novel anti-viral agent: Lamivudine", JOURNAL OF THE CHEMICAL SOCIETY, PERKIN TRANSACTIONS 2, CHEMICAL SOCIETY. LETCHWORTH, GB, vol. 12, 1 January 1997 (1997-01-01), pages 2653-2659, XP002432450, ISSN: 1472-779X

## Description

### FIELD OF THE INVENTION

The present invention relates to novel salts of Lamivudine and method for the preparation thereof. The present invention also relates to processes for the preparation of crystalline Lamivudine Form-I and Form-II from novel salts of Lamivudine.

### BACKGROUND OF THE INVENTION

Lamivudine of Formula I is an antiviral drug presently marketed by GlaxoSmithkline and is available as "EPIVIR", indicated for the treatment against retroviruses such as Human immuno deficiency virus (HIV), Hepatitis B virus (HBV) and Human T-Lymphotrophic virus (HTLV).

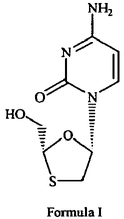

US 5047407 patent describes the preparation of (+)-(2*R*, *cis*)-4-amino-1-(2-hydroxymethyl-1, 3-oxathiolan-5-yl)-(1*H*)-pyrimidin-2-one (Lamivudine, 3TC), its antiviral activity and its use in pharmaceutical product.

US 6051709 patent discloses the process for the preparation of pure Lamivudine, in which Lamivudine isolated from its salicylate salt during the diastereoselective process development.

US 5905082 patent provides a process for the preparation of Lamivudine by enzymatic separation. This patent also discloses the existence of two polymorphic forms of Lamivudine *viz.,* **needle-shaped crystals (Form-1) and bipyramidal crystals (Form-II). It also discloses the processes for the preparation of Form-I and Form-II.** Further reference may be made to WO2009/069013, WO2009/037538, WO2008/114279, WO2007/119248 and Harris et al., "Journal of the Chemical Society, Perkin Transactions 2", vol. 12, 1997, pages 2653-2659.

The present invention provides novel acid addition salts of Lamivudine and process for the preparation of Lamivudine from these salts. In this process the recovery of acid is quantitative and quality of Lamivudine is better than salicylate process. The present process is cost effective and can be commercialized in large scale.

### SUMMARY OF THE INVENTION

In one aspect, the present invention provides novel acid addition salt of (+)-(2R, cis)-4-amino-1-(2-hydroxymethyl-1,3-oxathiolan-5-yl)-(1*H*)-pyrimidin-2-one, represented by Formula III.

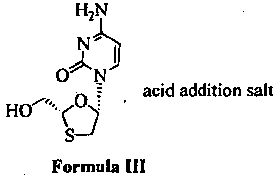

Wherein acid is selected from 3-hydroxy-2-naphthoic acid, L-pyroglutamic acid or 2-methoxybenzoic acid

In one aspect, the present invention provides novel (+)-(2*R*, cis)-4-amino-1-(2-hydroxymethyl-1,3-oxathiolan-5-yl)-(1*H*)-pyrimidin-2-one, 3-hydroxy-2-naphthoic acid salt represented by Formula IIIA.

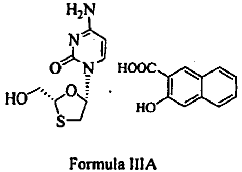

In another aspect, the present invention provides a process for the preparation of Formula III, comprising the steps of: a) reducing the compound of Formula II with metallic borohydride in solvent, b) adding 3-hydroxy-2-naphthoic acid or L-pyroglutamic acid or 2-methoxybenzoic acid, and c) isolating acid addition salt of Lamivudine of Formula III.

In another aspect, the present invention provides a process for the preparation of Lamivudine, comprising the steps of: a) reacting the compound of Formula III with a base in solvent and b) isolating the Lamivudine.

The entire process for the preparation of Lamivudine according to the present invention is as depicted in Scheme 1 below.

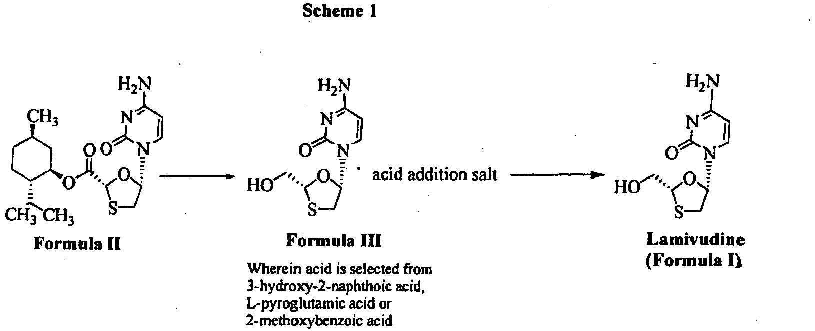

### BRIEF DESCRIPTION OF THE FIGURES

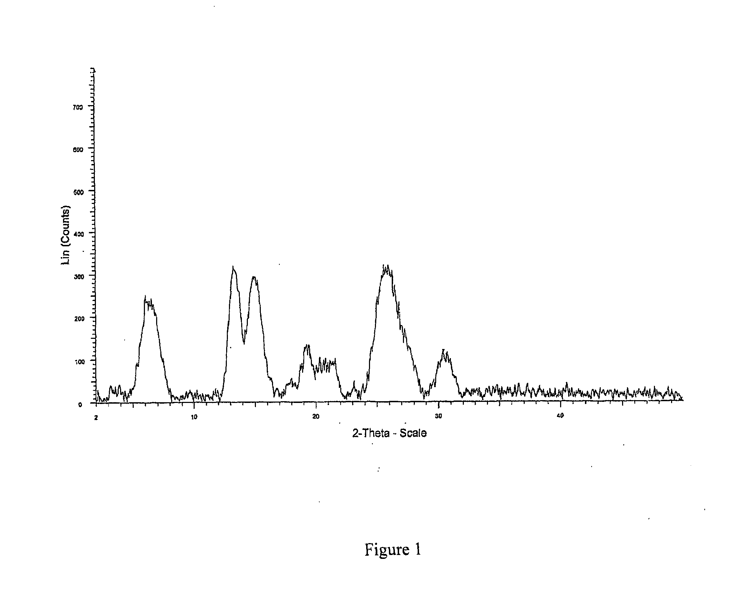

FIG. 1 is a representative X-ray diffraction pattern of (+)-(2*R*, *cis*)-4-amino-1-(2-hydroxymethyl-1, 3-oxathiolan-5-yl)-(1*H*)-pyrimidin-2-one 3-hydroxy-2-naphthoic acid salt (Formula IIIA).

### DETAILED DESCRIPTION OF THE INVENTION

### Definitions

The word "suspend" in the present invention may be partly soluble or insoluble. The word "treat" in the present invention may be contact or react.

In one embodiment, the present invention provides novel acid addition salt of (+)-(2*R*, cis)-4-amino-1-(2-hydroxymethyl-1,3-oxathiolan-5-yl)-(1*H*)-pyrimidin-2-one, represented by Formula III.

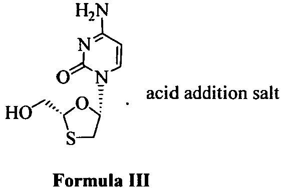

Wherein acid is selected from 3-hydroxy-2-naphthoic acid, L-pyroglutamic acid or 2-methoxybenzoic acid.

In another embodiment, the present invention provides a process for the preparation of acid addition salt of Lamivudine of Formula III,

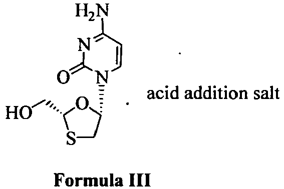

Wherein acid is selected from 3-hydroxy-2-naphthoic acid, L-pyroglutamic acid or 2-methoxybenzoic acid.

Comprising the steps of:
a) reducing the compound of Formula II with metallic borohydride in solvent, 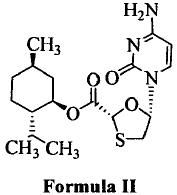
b) adding 3-hydroxy-2-naphthoic acid or L-pyroglutamic acid or 2-methoxybenzoic acid, and
c) isolating the acid addition salt of Lamivudine Formula III.

According to the present invention, compound of Formula II is suspended in solvent and reduced with metallic borohydride in presence of dipotassium hydrogen phosphate at 15-20°C. The resulting solution is added with an organic acid followed by addition of another solvent, heated to get a clear solution, cooled solution to room temperature and the obtained acid addition salt of Lamivudine is isolated by filtration.

The solvent used to dissolve the compound of Formula II is selected from ethanol, methanol, 1-propanol, 2-propanol, *N, N*-dimethylformamide, tetrahydrofuran, water or mixture thereof.

The metallic borohydride is selected from sodium borohydride, potassium borohydride or lithium borohydride.

The acid is selected from 3-hydroxy-2-naphthoic acid, methoxybenzoic acid or L-pyroglutamic acid. Another solvent is selected from ethanol, methanol, n-propanol, 2-propanol, acetone or methyl isobutyl ketone.

In another embodiment, the present invention provides a process for the preparation of Lamivudine comprising the steps of:
a) reacting the compound of Formula III with a base in solvent and
b) isolating the Lamivudine.

According to the present invention, compound of Formula III is suspended in solvent, treated with a base, heated the reaction mixture to get a clear solution and cooled to room temperature to crystallize the Lamivudine.

The solvent for the suspension of the compound of Formula III is selected from acetone, methyl isobutyl ketone, methanol, ethanol, 2-propanol, tetrahydrofuran, dioxane, ethyl acetate, water or mixture thereof. The base is selected from ammonia, triethylamine or Hunig's base.

In another embodiment, the present invention relates to 4-amino-1-(2*R*-hydroxymethyl-[1,3]-oxothiolane-5S-yl)-1*H*-pyrimidin-2-one 3-hydroxy-2-naphthoic acid salt, represented by Formula IIIA.

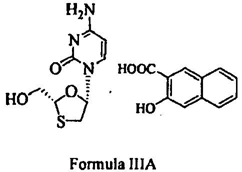

In another embodiment, the present invention relates to 4-amino-1-(2*R*-hydroxymethyl-[1,3]-oxothiolane-5*S*-yl)-1*H*-pyrimidin-2-one L-pyroglutamic acid salt, represented by Formula IIIB.

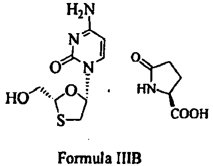

In another embodiment, the present invention relates to 4-amino-1-(2*R*-hydroxymethyl-[1, 3]-oxothiolane-5*S*-yl)-1*H*-pyrimidin-2-one 2-methoxybezoic acid salt, represented by Formula IIIC.

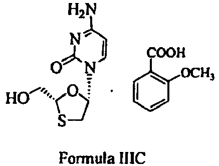

The following examples are provided for illustrative purposes only and are not intended to limit the scope of the invention in any way.

### EXPERIMENTAL SECTION

### EXAMPLE-1: Preparation of 4-amino-1-(2R-hydroxymethyl-[1,3]-oxothiolane-5S-yl)-1H-pyrimidin-2-one mononaphthylate (Lamivudine 3-hydroxy-2-naphthoic acid salt)

Ethanol (600 mL) was added to a solution of dipotassium hydrogen phosphate (137 g in 220 mL of water) and the mass was cooled to 18°C. (1'*R*, 2'*S*, 5'*R*)-Menthyl- 5*S*-cytosin-1"-yl-1,3-oxathiolane-2*R*-carboxylate (Formula II, 100 g) was added at 15-20°C and maintained for 1 h. A solution of sodium borohydride (48 g in 95 mL of 0.12N sodium hydroxide) was added drop wise by keeping temperature at 18-20°C and maintained for 4 h. After completion of the reaction, the layers were separated. The organic layerf pH was adjusted to 6.0-6.5 with 6N HCl (∼13 mL) and re-adjusted to pH 8.0 to 8.5 with 2N sodium hydroxide. Ethanol (~790 mL) was distilled out from the reaction mass under reduced pressure. The resultant mass was cooled to 30-35 °C, diluted with water (200 mL) under stirring and maintained for 15 min. The obtained aqueous layer was washed with toluene (100 mL) and charcoalised. 3-Hydroxy 2-naphthoicacid (48.92 g) and acetone (300 mL) were added to the aqueous layer and then heated to 60-65 °C to obtain a clear solution. The reaction mass was cooled to 10-15°C and allowed to stir for 2 h. The separated solid was filtered and washed with water (50 mL) followed by pre-cooled acetone (10 mL). The material was dried under vacuum at 45-50°C to yield Lamivudine 3-hydroxy-2-naphthoic acid salt in 90 g. Characterization of the title compound was confirmed by ¹H-NMR and ¹³C-NMR.

¹H-NMR (DMSO-d₆, 300 MHz, ppm)- δ 3.11-3.16 (dd, J=4.4 & 11.9 Hz, 1H, 6Ha), 3.43-3.48 (dd, J=5.4 & 12 Hz, 1H, 6Hb), 3.77 (d, J=4.5 Hz, 2H, 8), 5.21 (t, J=4.5 Hz, 1H, 7), 5.86 (d, J=7.5Hz, 1H, 3), 6.22 (t, J=5.0 Hz, 1H, 5), 7.23-7.26 (m, 1H, 14'), 7.30-7.35 (m, 1H, 14), 7.46-7.53 (m, 1H, 11), 7.74 (d, J=8.4 Hz, 1H, 13), 7.86-7.98 (m, 1H, 13'), 7.97 (d, J=7.8 Hz, 1H, 4), 8.48-8.51 (m, 1H, 16).

¹³C-NMR (DMSO-d₆, 75 MHz, ppm)- δ 37.03(6C), 62.19 (8C), 93.96(3C), 86.65(7C), 87.43(5C), 110.07(11C), 118.37(9C), 123.38(14'C), 125.76(13C), 126.64(15C), 128.18(16C), 129.05(14C), 131.96(13'C), 136.88(12C), 142.92(4C), 150.93 (1C), 157.12(10C), 162.65(2C), 172.44(17C).

### EXAMPLE-2: Preparation of 4-amino-1-(2R-hydroxymethyl-[1, 3]-ozothiolane-5S-yl)-1H-pyrimidin-2-one mononaphthylate (Lamivudine 3-hydroxy-2-naphthoicacid salt)

Ethanol (600 mL) was added to a solution of dipotassium hydrogen phosphate (83.3 g in 220 mL of water) and the mass was cooled to 18°C. (1'*R*, 2'*S*, 5'*R*)-Menthyl-5*S*-cytosin- 1"-yl-1, 3-oxathiolane-2*R*-carboxylate (Formula II, 100 g) was added at 12-18°C and maintained for 1 h. A solution of sodium borohydride (20 g in 95 mL of 0.5 g of sodium hydroxide) was added drop wise by keeping temperature at 12-18 °C and maintained for 4 h. After completion of the reaction, the layers were separated. The organic layer pH was adjusted to 6.0-6.5 with 6N HCl (~13 mL) and re-adjusted to pH 8.0 to 8.5 with 2N sodium hydroxide. Ethanol (450 mL) was distilled out from the reaction mass under reduced pressure. Then resultant mass was cooled to 30-35 °C, diluted with water (200 mL) under stirring. The obtained aqueous layer was washed with toluene (100 mL) and charcoalised. 3-Hydroxy2-naphthoicacid (49.4 g) was added to aqueous layer, heated to 82-88 °C to obtain a clear solution and charcoalised. Reaction mass diluted with purified water (100 mL), heated to 82-88°C and cooled to 10-15°C. The separated solid was filtered and washed with chilled purified water (50 mL). The wet cake was added into purified water (300 mL) and stirred for 60 min. at 30-35°C. Further, cooled to 10-15°C and allowed to stir for 2 h. The separated solid was filtered and washed with chilled purified water (50 mL). The material was dried at 71-75°C to yield Lamivudine 3-hydroxy-2-naphthoicacid salt in 90g. Diastereomer impurity: <0.5%.

### EXAMPLE 3: Preparation of 4-amino-1-(2R-hydroxymethyl-[1, 3]-oxothiolane-5S-yl) 1H-pyrimidin-2-one L-Pyroglutamate (Lamivudine L-pyroglutamate)

Methanol (90 mL) and L-pyroglutamic acid (34.9 g) was added to the aqueous layer of Ex-1 (after toluene washings), heated to 60-65 °C to obtain a clear solution and maintained for 30 min. The reaction mass was cooled to 10 -15°C and allowed to stir for 2 h. The separated solid was filtered, washed with methanol (10 mL) and dried under vacuum at 45-50°C to afford Lamivudine L-pyroglutamate in 90 g.

### EXAMPLE-4: Preparation of preparation of 4-amino-1-(2R-hydroxymethyl-[1, 3]-oxothiolane-5S-yl) 1H-pyrimidin-2-one 2-methoxybezoicacid salt (Lamivudine 2-methoxybezoicacid salt)

2-Methoxy benzoic acid (39.62 g) was added to the aqueous layer (700 mL) from Example-1 (after toluene washings) and heated to 60-65°C to obtain a clear solution. The reaction mass was stirred for 30 min and diluted with water (100 mL) at 60-65°C. The reaction mass was cooled to 0-5°C and allowed to stir for 2 h. The separated solid was filtered, washed with chilled methanol (50 mL) and dried under vacuum at 45-50°C to get Lamivudine 2-methoxy benzoic acid in 75 g.

### EXAMPLE-5: Preparation of 4-amino-1-(2R-hydroxymethyl-[1,3]-oxothiolane-5S-yl) 1H-pyrimidin-2-one (Lamivudine) from Lamivudine 3-hydroxy-2-naphthoic acid salt.

Lamivudine naphthylate (90 g) was suspended in 2% aqueous acetone (400 mL) and triethylamine (43.59 g) was added at 25-30 °C. The reaction mass was heated to 40-45°C and maintained for 30 min. The reaction mass was cooled to 25-30 °C over period of 60 min. to crystallize the material. The separated solid was filtered, washed with acetone (20 mL) and dried under vacuum at 45-50°C to obtained Lamivudine in 46 g.

### EXAMPLE-6: Preparation of 4-amino-1-(2R-hydroxymethyl-[1,3]-oxothiolane-5S-yl) 1H-pyrimidin-2-one (Lamivudine) from Lamivudine 3-hydroxy-2-naphthoicacid salt.

Lamivudine naphthylate (90 g) was suspended in a mixture of acetone (500 mL), methanol (50 mL) and triethylamine (48.4 g) at 25-30°C. The reaction was heated to 50-55°C and maintained for 2 h. The reaction was cooled to 21-25°C over a period of 3 h. to crystallize the material. The separated solid was filtered, washed with acetone (50 mL). The wet cake was added into acetone (300 mL), heated to 50-55°C and maintained for 60 min. The reaction was cooled to 21-25°C over a period of 60 min. to crystallize the material. The separated solid was filtered, washed with acetone (50 mL). The material was dried at 40-45°C to yield Lamivudine in 45g. Diastereomer impurity: <0.3%.

### EXAMPLE-7: Preparation of 4-amino-1-(2R-hydroxymethyl-[1,3]-oxothiolane-5S-yl) 1H-pyrimidin-2-one (Lamivudine) from Lamivudine L-pyroglutamate.

Lamivudine L-pyroglutamate (80 g) was suspended in aqueous acetone (300 mL) and triethylamine (44.64 g) was added at 25-30 °C. The reaction mass was heated to 40-45 °C maintained for 30 min. the reaction mass was cooled to 25-30 °C over a period of 60 min. to crystallize the material. The separated solid was filtered, washed with acetone (10 mL) and dried under vacuum at 45-50°C to yield Lamivudine in 48 g.

### EXAMPLE-8: Preparation of 4-amino-1-(2R-hydroxymethyl-[1,3]-oxothiolane-5S-yl) 1H-pyrimidin-2-one (Lamivudine) from Lamivudine methoxy benzoate

Lamivudine methoxy benzoate (90 g) was suspended in aqueous acetone (300 mL) and triethylamine (47.66 g) was added at 25-30 °C. The reaction mass was heat to 40-45 °C and maintained for 30 min. The reaction mass was cooled to 25-30 °C over period of 60 min. to crystallize the material. The separated solid was filtered, washed with acetone (20 mL) and dried under vacuum at 45-50°C to afford Lamivudine in 50 g.

## Claims

1. A process for the preparation of an acid addition salt of Lamivudine Formula III 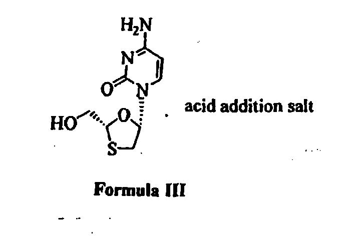 wherein the acid is selected from the group consisting of 3-hydroxy-2-naphthoic acid, L-pyroglutamic acid and 2-methoxybenzoic acid;
comprising the steps of
a) reducing the compound of Formula II with metallic borohydride in a solvent, 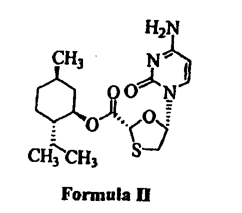
b) adding 3-hydroxy-2-naphthoic acid, L-pyroglutamic acid or 2-methoxybenzoic acid, and
c) isolating the acid addition salt of Lamivudine Formula III.

2. The process according to claim 1, wherein the solvent is selected from the group consisting of ethanol, methanol, 1-propanol, 2-propanol, N,N-dimethylformamide, tetrahydrofuran, water and mixtures thereof.

3. The process according to claim 1, wherein the metallic borohydride is selected from the group consisting of sodium borohydride, potassium borohydride and lithium borohydride.

4. A process for the preparation of Lamivudine comprising the steps of
a) reacting the compound of Formula III with a base in a solvent and
b) isolating Lamivudine.

5. The process according to the claim 4, wherein the solvent is selected from the group consisting of acetone, methyl isobutyl ketone, methanol, ethanol, 2-propanol, tetrahydrofuran, dioxane, ethyl acetate, water and mixtures thereof.

6. The process according to the claim 4, wherein the base is selected from the group consisting of ammonia, triethylamine and Hunig's base.

7. A compound of Formula

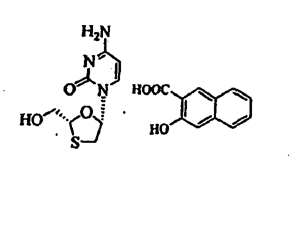

8. A compound of Formula

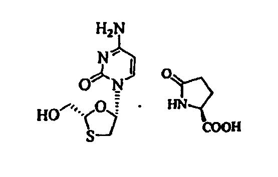

9. A compound of Formula

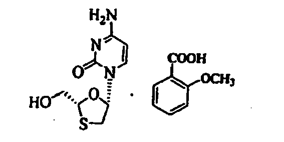

## Patentansprüche

1. Verfahren zur Herstellung eines Säureadditionssalzes von Lamivudin mit der Formel III, 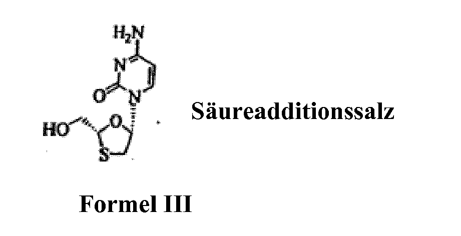 wobei die Säure aus einer Gruppe ausgewählt wird, die sich aus Folgendem zusammensetzt: 3-Hydroxy-2-Naphthoesäure, L-Pyroglutaminsäure und 2-Methoxybenzoesäure; das folgende Schritte umfasst:
a) Reduktion der Verbindung aus Formel II mit metallischem Borhydrid in einem Lösungsmittel, 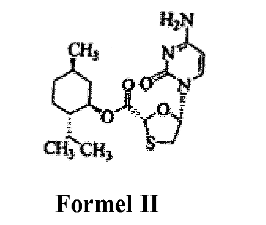
b) Hinzufügen von 3-Hydroxy-2-Naphthoesäure, L-Pyroglutaminsäure und 2-Methoxybenzoesäure und
c) Isolieren des Säureadditionssalzes von Lamivudin mit der Formel III.

2. Verfahren nach Anspruch 1, wobei das Lösungsmittel aus einer Gruppe ausgewählt wird, die sich aus Folgendem zusammensetzt: Ethanol, Methanol, 1-Propanol, 2-Propanol, N,N-Dimethylformamid, Tetrahydrofuran, Wasser und Gemischen davon.

3. Verfahren nach Anspruch 1, wobei das metallische Borhydrid aus einer Gruppe ausgewählt wird, die sich aus Folgendem zusammensetzt: Natriumborhydrid, Kaliumborhydrid und Lithiumborhydrid.

4. Verfahren zur Herstellung von Lamivudin, das folgende Schritte umfasst:
a) Reagierenlassen der Verbindung aus Formel III mit einer Base in einem Lösungsmittel und
b) Isolieren von Lamivudin.

5. Verfahren nach Anspruch 4, wobei das Lösungsmittel aus einer Gruppe ausgewählt wird, die sich aus Folgendem zusammensetzt: Aceton, Methylisobutylketon, Methanol, Ethanol, 2-Propanol, Tetrahydrofuran, Dioxan, Ethylacetat, Wasser und Gemischen davon.

6. Verfahren nach Anspruch 4, wobei die Base aus einer Gruppe ausgewählt wird, die sich aus Folgendem zusammensetzt: Ammoniak, Triethylamin und Hünig-Base.

7. Eine Verbindung mit der Formel

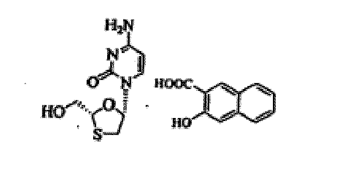

8. Eine Verbindung mit der Formel

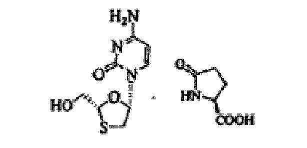

9. Eine Verbindung mit der Formel

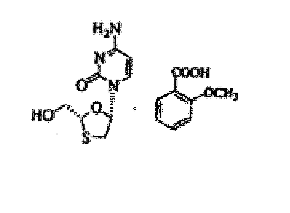

## Revendications

1. Procédé de préparation d'un sel d'addition acide de Lamivudine de formule III 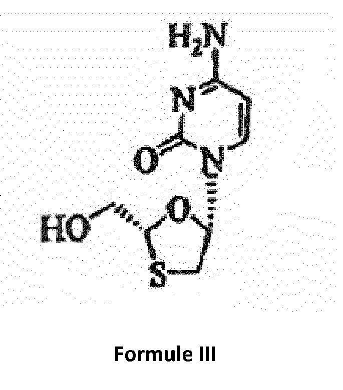 dans lequel l'acide est sélectionné parmi les composés du groupe consistant à l'acide 3-hydroxy-2-naphthoïque, L-pyroglutamique et l'acide 2-méthoxybenzoïque ;
comprenant les étapes de :
a) Réduction du composé de formule Il par un borohydrure métallique dans un solvant, 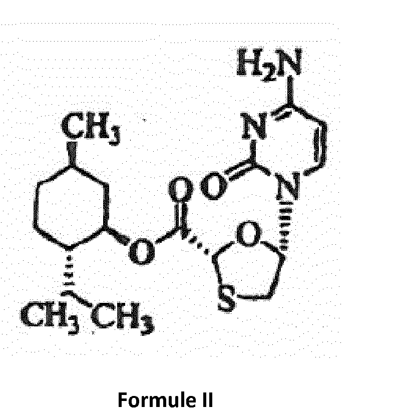
b) Ajout de l'acide 3-hydroxy-2-naphtoïque, L-pyroglutamique et l'acide 2-méthoxybenzoïque, et
c) L'isolation/Séparation du sel d'addition acide de Lamivudine de formule III.

2. Procédé selon revendication 1, dans lequel le solvant est choisi parmi les composés du groupe consistant à l'éthanol, le méthanol, le 1-propanol, le 2-propanol, le N,N-diméthylformamide, le tétrahydrofurane, l'eau et mélanges de ceux-ci.

3. Procédé selon la revendication 1, dans lequel le borohydrure métallique est sélectionné parmi les composés du groupe consistant au borohydrure de sodium, le borohydrure de potassium et le borohydrure de lithium.

4. Un procédé de préparation de Lamivudine comprenant les étapes de :
a) Faire réagir le composé de formule III avec une base dans un solvant et
b) Isoler/séparer le composé de Lamivudine

5. Procédé selon la revendication 4, dans lequel le solvant est sélectionné parmi les composés du groupe consistant à l'acétone, la cétone méthyl-isobutyl, le méthanol, l'éthanol, le 2-propanol, le tétrahydrofurane, le dioxane, l'acétate d'éthyl, l'eau et mélanges de ceux-ci.

6. Procédé selon la revendication 4, dans lequel la base est sélectionnée parmi les composés du groupe consistant à l'ammoniac, la triéthylamine et la base de Hunig.

7. Composé de formule :

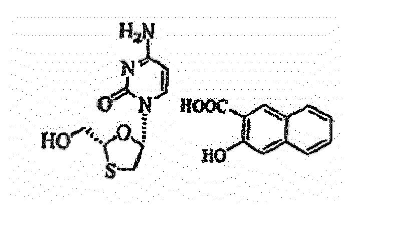

8. Composé de formule :

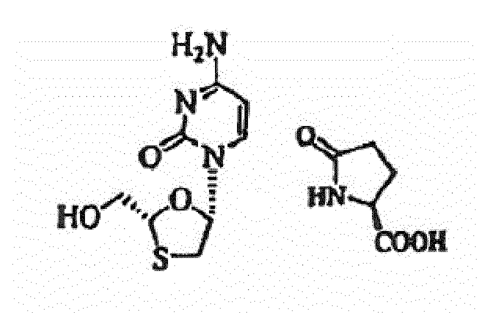

9. Composé de formule :
